(19) Europäisches Patentamt
European Patent Office
Office européen des brevets

(11) Numéro de publication: **0 189 611**
**B1**

(12) # FASCICULE DE BREVET EUROPEEN

(45) Date de publication du fascicule du brevet: **12.04.89**

(51) Int. Cl.⁴: **B 01 D 15/08**, B 01 D 15/02, C 07 K 3/20, A 23 J 1/20

(21) Numéro de dépôt: **85202068.4**

(22) Date de dépôt: **13.12.85**

(54) **Procédé pour la séparation chromatographique de macromolécules biologiques.**

(30) Priorité: **04.01.85 FR 8500463**

(43) Date de publication de la demande: **06.08.86 Bulletin 86/32**

(45) Mention de la délivrance du brevet: **12.04.89 Bulletin 89/15**

(84) Etats contractants désignés: **AT BE CH DE FR GB IT LI LU NL SE**

(56) Documents cités:
**EP-A- 0 086 068**
**DE-A- 2 638 764**
**FR-A- 2 105 032**
**GB-A- 1 148 661**
**GB-A- 1 422 412**
**US-A- 4 284 511**

(73) Titulaire: **Etablissement Public dit: CENTRE NATIONAL DE LA RECHERCHE SCIENTIFIQUE (CNRS), 15, Quai Anatole France, F-75007 Paris (FR)**

(72) Inventeur: **Sanchez, Victor, 1, rue Léonard de Vinci, F-31520 Ramonville Saint-Agne (FR)**
Inventeur: **Biscans-Milcan, Béatrice, 7, rue de la Tramontane, F-31320 Escalquens (FR)**
Inventeur: **Couderc, Jean-Pierre, 26, rue Rouvières, F-31100 Toulouse (FR)**
Inventeur: **Riba, Jean-Pierre, 5, rue de Bougainville, F-31400 Toulouse (FR)**

(74) Mandataire: **Barre, Philippe, Cabinet Barre-Gatti-Laforgue 95 rue des Amidonniers, F-31069 Toulouse Cédex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

L'invention concerne un procédé permettant de séparer, par chromatographie c'est-à-dire par un processus d'adsorption dont le caractère essentiel réside dans sa sélectivité – des macromolécules biologiques de poids moléculaire élevé, telles que protéines, enzymes, toxines, anticorps... en vue d'obtenir des solutions enrichies dans une ou des macromolécules recherchées.

Certaines molécules biologiques, dites macromolécules biologiques, sont caractérisées par des masses moléculaires très élevées (supérieures à 5000) et une tendance à se dénaturer, provoquant la perte de leur activité biologique ou de leur capacité fonctionnelle (exemple: dénaturation des protéines en peptides); ces propriétés limitent le type de procédés de séparation utilisables à l'échelle industrielle pour les purifier (précipitation, ultrafiltration et chromatographie). Les procédés de chromatographie (adsorption sélective) constituent actuellement les techniques préparatives les plus spécifiques et les mieux adaptées pour la production à échelle industrielle de macromolécules biologiques de pureté élevée.

Ces procédés sont mis en œuvre par percolation d'une solution contenant les macromolécules biologiques à travers un lit fixe de résines chromatographiques spécifiques aptes à engendrer une adsorption sélective de celles-ci. Dans le cas où la (ou les) macromolécule recherchée est fixée sur la résine, une élution de cette dernière avec une solution de pH ou de force ionique appropriée permet de la séparer et de l'obtenir purifiée et concentrée. Dans le cas où la macromolécule recherchée reste dans la solution traitée, (les autres macromolécules étant fixées sur la résine) on obtient directement la séparation recherchée.

Cette technique de chromatographie en lit fixe et ses performances sont bien connues dans le secteur des biotechnologies et on pourra notamment se reporter aux documents suivants qui en fournissent des exemples:

- «Method of Plasma Protein fractionnation», by J.M. CURLING, Académic Press, 1980 p. 149–160,
- «Protein recovery by ion exchange, by D.T. JONES Bsc, Food Processing Industry, April 1975 p. 21, 23,
- «Nouveau procédé de valorisation du lactosérum» par B. MIRABEL, Rhône-Poulenc Industries, Informations chimie» n° 175, mars 1978 p. 105–109,
- Brevets FR 2 321 932 et 2 359 634 décrivant de nouvelles résines échangeuses d'ions pour la séparation de protéines.

Toutefois, ces procédés de séparation chromatographique en lit fixe présentent des défauts dont le plus grave réside dans l'apparition progressive d'un colmatage du lit; ce colmatage est essentiellement provoqué par les impuretés solides en suspension dans la solution et, souvent, par la précipitation de certains composants de celle-ci. Les conséquences en sont extrêmement graves sur le plan industriel. Pour conserver un débit de percolation constant au cours du temps, la pression d'entrée de la solution doit augmenter jusqu'à prendre des valeurs de l'ordre de 3 à 4 fois supérieures aux valeurs nominales, ce qui entraîne des difficultés techniques considérables ou même peut être inacceptables dans certaines applications. De plus, le nettoyage cyclique des résines chromatographiques est une opération extrêmement délicate en pratique et conduit généralement à les laver dans des enceintes séparées, ce qui fait obstacle à un fonctionnement continu des installations et impose des manipulations délicates et onéreuses. Il est à noter que certaines résines chromatographiques, généralement de nature minérale, ont des supports fragiles qui se cassent au cours des nettoyages pour donner des fines susceptibles ensuite d'obstruer certaines zones du lit, et que d'autres résines chromatographiques, généralement de nature organique, ne peuvent pas être nettoyées lorsque leurs mailles sont colmatées et doivent être rebutées. Dans certains cas, le colmatage entraîne une prise en masse du lit dans son ensemble et un nettoyage n'est plus possible. Une autre conséquence extrêmement préjudiciable du colmatage réside dans la dérive de la capacité de fixation du lit de résine après plusieurs nettoyages: quelles que soient les précautions prises, une certaine proportion d'impuretés reste fixée sur la résine chromatographique et, peu à peu, la capacité de fixation de celle-ci décroît jusqu'à ce qu'il devienne nécessaire de la rebuter.

Ces difficultés sont bien connues des spécialistes de la chromatographie biologique et, dans la plupart des cas, elles obligent à réaliser une épuration approfondie des solutions avant leur traitement. Cette épuration est un facteur d'augmentation considérable du coût du procédé et limite l'application de celui-ci à l'obtention de produits de valeur élevée (médicament...) écartant son utilisation dans un grand nombre d'applications où les macromolécules produites présentent une plus faible valeur (traitement de certains produits naturels d'origine biologique ou de sous-produits d'industries notamment agro-alimentaire).

Un autre défaut des procédés de chromatographie en lit fixe réside dans la brièveté de la durée de vie des résines qui sont assez rapidement détruites par les contraintes provoquées par leur tassement et les divers traitements intermédiaires de lavage et décolmatage qu'elles subissent.

Actuellement, les problèmes évoqués ci-dessus demeurent non résolus ou résolus au prix d'une augmentation importante des coûts de mise en œuvre dans le cas des macromolécules biologiques; compte tenu de l'importance économique de ce type de traitements qui touchent aussi bien le secteur alimentaire, que le secteur pharmaceutique, vétérinaire, etc..., de nombreuses études ont été effectuées pour tenter de minimiser les conséquences préjudiciables résumées précédemment, afin de pouvoir profiter pleinement des performances des procédés chromatographiques pour séparer des macromolécules biologiques. Ainsi certains auteurs ont préconisé une mise en œuvre

en lit tassé mobile, constitué par un lit qui est déplacé couche par couche par prélèvement de la résine à une extrémité avec réinsertion à l'autre extrémité après lavage et nettoyage (D.T. Jones Bsc, «Protein recovery by ion exchange»). Toutefois, aucune solution satisfaisante d'application générale, n'a jusqu'à présent été trouvée, certains défauts graves subsistant toujours, tels par exemple que la nécessité de nettoyage, la dérive de la capacité de fixation et l'usure des résines chromatographiques.

Par ailleurs, dans d'autres secteurs techniques et en particulier dans celui des séparations minérales, on réalise depuis longtemps des chromatographies en lit fluidisé, qui évitent le colmatage et réduisent l'usure des résines:

– séparation d'ions métalliques: «A continuous ion exchange column, by Turner and Church, Trans. Instn Chem. Engrs, Vol 41, 1963 p. 283–288»,

– extraction d'uranium: «Assessment of Fluidised Bed Ion Exchanqe Equipment, Michaël J. Slater, J. Appl. Chem. Biotechnol. 1975, vol. 25 p. 367–378».

Ces procédés de chromatographie en lit fluidisé sont connus depuis plus de 20 ans pour séparer des molécules de petite taille (ayant une bonne stabilité). Toutefois, les biotechniciens n'ont jamais tenté de les utiliser pour la chromatographie des macromolécules biologiques de poids moléculaire supérieur à 5000, car cette technique a toujours paru impossible à adapter à ce type de traitement pour diverses raisons plus ou moins objectives. Certaines tiennent, dans l'esprit des spécialistes, au type de résines chromatographiques spécifiques des macromolécules biologiques (trop faible granulométrie, densité trop proche de celle de l'eau, nature physique inadaptée), qu'il serait impossible de fluidiser sans entraînement des particules dans le flux. D'autres raisons tiennent à la cinétique de fixation des macromolécules biologiques qui, en raison notamment des volumes intersticiels trop importants, subirait une chute d'efficacité en lit fluidisé rendant le procédé inopérant. D'autres raisons tiennent aux vitesses infinitésimales auxquelles il serait nécessaire d'entraîner la solution à travers une couche fluidisée (inadaptation à des productions industrielles)...

Ainsi, déjà en 1980, le préjugé existant était suffisamment ancré dans l'esprit de l'homme de l'art pour qu'un éminent biotechnicien écrive dans la publication déjà évoquée «Methods of Plasma Protein Frationation, J.M. Curling, p. 152» (traduction):

«Nous avons choisi une colonne en lit fixe plutôt qu'une colonne en lit fluidisé. La fluidisation impose un flux de filtration ascendant et de grosses particules assez denses pour se maintenir en équilibre sous ces forces ascendantes. Mais les gels disponibles pour le fractionnement des protéines sont rarement plus denses que l'eau et donneraient des suspensions extrêmement diluées même à faible vitesse de flux. Au surplus, en raison de la lente diffusion des macromolécules, la taille des grains et celle des volumes intersticiels doivent être aussi petits que possible dans le but d'augmenter la probabilité de pénétration de l'élément liquide dans le volume interne poreux. Le lit fixe conduit à un volume de colonne minimal et économise la consommation d'eau. Tous ces arguments ont des conséquences financières qui ne sont pas à négliger lorsque l'on choisit la méthodologie générale. Avec toutes ces exigences, il est évident que la mise en œuvre des méthodes chromatographiques dans l'industrie biologique demande des support spéciaux ayant des qualités mécaniques exceptionnelles. Ils doivent être stérilisables quand ils sont utilisés dans une colonne sous la forme la plus compacte et ils doivent permettre des vitesses de flux très élevées quand il y a plusieurs centaines ou milliers de litres de solution à traiter chaque jour».

Il est à noter que deux brevets très antérieurs (brevet GB n° 1 148 661 du 09.05.66 et brevet FR n° 2 105 032 du 17.09.70) évoquent, l'un une chromatographie de molécules biologiques en lit fixe, fluidisé ou en colonne ouverte, l'autre un échange ionique en lit fluidisé pour purifier une eau dure ou un sel d'uranium en citant comme application possible la purification de substances biologiques. Toutefois l'on comprend que ces brevets aient été inaptes à empêcher que se forme le préjugé ci-dessus évoqué. En effet, le premier brevet qui vise un traitement en conditions turbulentes quelle que soit la forme du lit – fixe, fluidisé ou ouvert – décrit simplement deux exemples de chromatographies de molécules biologiques qui portent tous deux sur des molécules de petites tailles: codéine et atropine; par ailleurs, ce brevet fait, dans sa partie générale, référence à une liste d'applications possibles: hormones, vitamines, alkaloïdes, antibiotiques, lesquelles sont toutes de petites molécules à part certaines hormones. Le second brevet fournit comme exemples uniquement la purification d'une eau dure pour en éliminer les sels et la purification d'un sel d'uranium; ce brevet évoque, dans son préambule général, l'application du processus d'échange ionique sur des substances biologiques et l'on sait que les simples processus d'échanges ioniques ne sont applicables qu'aux molécules de petites tailles (contrairement aux processus chromatographiques qui font intervenir des résines chromatographiques sélectives de type spécifique).

La présente invention se propose d'améliorer la technique actuelle de séparation chromatographique des macromolécules biologiques dans le secteur des biotechnologies. Elle vise la chromatographie de molécules de fortes masses moléculaires (supérieures à 5000), telles que protéines, enzymes, toxines, anticorps.

Un objectif essentiel de la présente invention est en particulier de permettre, pour ces macromolécules biologiques, la mise en œuvre de séparations chromatographiques échappant au défaut le plus grave de ces séparations consistant dans le colmatage des lits.

Un autre objectif lié au précédent est de réduire considérablement l'usure des particules de résines

constitutives des lits.

Un autre objectif est d'autoriser une mise en œuvre en continu des séparations.

Un autre objectif est de rendre la séparation chromatographique des macromolécules biologiques économiquement applicable à l'échelle industrielle à un très grand nombre de corps de masse moléculaire élevée, notamment en vue d'assurer la valorisation, soit de sous-produits protéïques d'industries agro-alimentaires, soit de produits naturels tels que le lait, le sang, le plasma ou le sérum sanguin, soit de produits synthétiques.

A cet effet, le procédé visé par l'invention pour effectuer une séparation chromatographique de macromolécules biologiques de poids moléculaire supérieur à 5000 contenues dans une solution est du type consistant à amener la solution au contact d'un lit de résines chromatographiques sélectives, spécifiques des macromolécules à séparer en vue de réaliser une adsorption sélective; selon la présente invention, le procédé se caractérise en ce que:

– l'on utilise une résine chromatographique ayant une granulométrie moyenne $G_m$ et une masse volumique $M_v$ dont les valeurs sont situées dans la zone z (non hachurée) du diagramme de la figure 1,

– l'on dispose ladite résine chromatographique dans une colonne comprenant au moins un étage pourvu à sa base d'un système perforé de distribution présentant les caractéristiques suivantes:

– pourcentage de sections ouvertes compris entre 0,1% et 10%,

– diamètre moyen des sections ouvertes supérieur à environ 300 µm,

– diamètre moyen des sections ouvertes compris entre 2 $G_m$ et 20 $G_m$,

– et l'on alimente le ou chaque étage de la colonne à travers son système de distribution au moyen de la solution de manière à fluidiser le lit de résine chromatographique, en ajustant le débit de la solution de façon à obtenir dans la colonne une vitesse linéaire du liquide (ramenée à la section droite totale) comprise entre 1,5 $V_{mf}$ et 12 $V_{mf}$, où $V_{mf}$ est la vitesse minimale de fluidisation de la résine.

La granulométrie moyenne $G_m$ de la résine est définie de façon usuelle par la formule $G_m = \frac{\Sigma xi\ Gi}{\Sigma xi}$ où xi est la masse de la fraction des particules ayant la granulométrie Gi.

Ainsi, de façon inattendue par rapport aux enseignements de l'art antérieur, on a pu constater qu'il était possible de réaliser une séparation chromatographique de macromolécules biologiques, par adsorption sélective en lit fluidisé, en combinant les conditions sous-évoquées relatives à la résine chromatographique et à la distribution de la solution (structure des distributeurs, débit d'alimentation). Comme les exemples fournis plus loin le montreront, de façon surprenante, la mise en œuvre en lit fluidisé ne réduit pas les capacités d'échange de la résine chromatographique et les transferts sélectifs de matières demeurent globalement sensiblement similaires à ceux obtenus en lit fixe.

De préférence, on choisira une résine chromatographique de granulométrie moyenne $G_m$ comprise entre 75 et 300 µm correspondant sur le diagramme de la figure 1 à la bande horizontale comprise entre la courbe limite C et les deux droites $D_1$ et $D_2$. Il s'est avéré qu'il y avait intérêt à ce que la dispersion de la taille des grains ne soit pas trop grande et un tamisage préalable desdites résines sera préférentiellement effectué pour limiter la dispersion à environ 50% autour de la valeur moyenne $G_m$.

Par ailleurs, selon un mode de mise en œuvre préféré, la résine chromatographique est disposée dans une colonne sensiblement verticale comprenant entre 2 et 5 étages superposés, parcourus successivement par la solution, le soutirage de ladite solution étant effectué par surverse en partie haute de l'étage supérieur, chaque étage (à l'exception de ce dernier) étant délimité par deux systèmes perforés de distribution, l'un distribuant la solution à la base de l'étage considéré, l'autre distribuant la solution vers l'étage situé immédiatement au-dessus.

On a pu constater que, pour une quantité globale de résine chromatographique, le fractionnement en lit fluidisé à plusieurs étages améliore considérablement les performances de séparation dans un temps donné et permet de se rapprocher des performances obtenues en lit fixe. Ce phénomène peut s'expliquer par le fait que l'on tend ainsi à recréer un gradient de concentration (favorisant la séparation) qui existe naturellement en lit fixe mais non en lit fluidisé à un seul étage.

De la sorte, le procédé de l'invention permet à la fois d'atteindre sensiblement les performances des procédés en lit fixe, tout en étant affranchi des graves défauts de ce type de lit: colmatage et nécessité de nettoyage, détérioration des résines provoquée par le tassement et les nettoyages. En effet, le lit fluidisé autorise le libre passage des impuretés de la solution sans risque de colmatage; aucun nettoyage n'est nécessaire de sorte que la durée de vie des résines est considérablement accrue.

De plus, le procédé peut être mis en œuvre en continu en raison de l'absence des contraintes créées par les nettoyages: la résine chromatographique peut être amenée à circuler en continu de haut en bas dans la colonne, avec une alimentation continue en résine en partie haute et un soutirage continu en partie basse, la séparation en lit fluidisé étant effectuée en continu dans la colonne, avec exécution concomitante d'opérations de lavage, élution et lavage à l'extérieur de la colonne.

Il convient de ne pas confondre les opérations classiques de lavage qui consistent en un simple rinçage à l'eau distillée ou autre solution, ou opération d'élution qui consiste en un passage d'une solution de régénération, avec les opérations de nettoyage ou décolmatage requièrent dans les procédés connus un traitement mécanique énergique des résines et sont à l'origine de leur détérioration et des difficultés de mise en œuvre en continu.

La circulation de la résine chromatographique entre deux étages superposés, peut être assurée par un tube de passage, agencé de façon à permettre à la résine de circuler par surverse dans ledit tube de l'étage du dessus à l'étage du dessous.

De plus, une ou des chicanes sont préférentiellement disposées à chaque étage en vue d'assurer un renouvellement complet de la résine de l'étage, sans apparition de zones mortes.

Bien entendu, le procédé de l'invention peut, le cas échéant, être mis en œuvre en discontinu: la résine chromatographique est alors enfermée à chaque étage de la colonne en l'absence de circulation d'un étage à l'autre, la séparation en lit fluidisé étant effectuée par cycles de fixation sélective, lavage, élution et lavage dans la colonne. Ce mode de mise en œuvre reste avantageux par rapport aux séparations en lit fixe pour les raisons déjà évoquées: en particulier, il n'est pas nécessaire de démonter la colonne pour des décolmatages.

Dans les deux cas de mise en œuvre (continu, discontinu) la colonne est avantageusement alimentée de sorte que le débit de solution à l'intérieur de celle-ci, soit sensiblement constant au moins pendant les phases de fixation sélective, la variation de ce débit par rapport à sa valeur nominale étant inférieure à $\mp 5\%$.

Cette disposition garantit une fluidisation stable de la résine chromatographique sans risque d'entraînement accidentel de grains.

Le procédé de l'invention est applicable de façon générale pour la séparation de toute macromolécule biologique de poids moléculaire supérieur à 5000; l'homme du métier est apte, dans chaque application, à choisir une résine chromatographique sélective (conditions de masse volumique et granulométrie fournies par le diagramme de la figure 1) et à réaliser un système perforé de distribution convenant à la granulométrie de ladite résine (conditions déjà évoquées de diamètres des sections ouvertes).

En particulier, le procédé de l'invention peut être appliqué à la séparation chromatographique des protéines d'un lactosérum; la résine utilisée peut notamment être une résine du type «résine Sphérosil» vendue sous ce nom par la Société «Rhône Poulenc» et décrite dans le brevet français n° 2 321 932; cette résine répond aux spécifications fournies par le diagramme de la figure 1.

La description qui suit fournit, à titre non limitatif, des exemples de mise en œuvre du procédé de l'invention réalisés, d'une part, dans une installation discontinue (exemples 1 et 2), d'autre part, dans une installation continue (exemple 3); les figures de dessins annexés représentent ces installations:

– la figure 1 déjà évoquée est un diagramme donnant les spécifications des résines appropriées;

– la figure 2 est une vue schématique d'une installation discontinue pour la mise en œuvre du procédé,

– la figure 3 est une coupe verticale de détail de la colonne de ladite installation,

– la figure 4 est une vue schématique d'une installation continue,

– la figure 5 est une coupe verticale de détail de la colonne de ladite installation,

– la figure 6 en est une coupe transversale par un plan horizontal AA',

– les figures 7, 8 et 9 sont des diagrammes donnant, en fonction du temps, l'évolution des concentrations de sortie de la solution, respectivement pour chacun des exemples 1 à 3.

L'installation représentée à titre d'exemple aux figures 2 et 3, est destinée à assurer une séparation chromatographique de macromolécules biologiques selon un processus de fonctionnement cyclique, alternant une phase de fixation, une phase de lavage, une phase d'élution et une nouvelle phase de lavage.

Au cours de ces différentes phases, la résine chromatographique spécifique est enfermée dans une colonne verticale 1 à plusieurs étages, tels que 3 ou 4. Trois ou quatre étages pourront être prévus en pratique.

La colonne 1 comprend une embase 2 qui permet de distribuer les solutions vers l'étage inférieur 3, à travers un système perforé de distribution. Ce système est identique pour tous les étages et à été représenté en 5 à échelle dilatée à la figure 3.

Il comprend deux plaques cylindriques perforées 6 et 7 (ou le cas échéant un nombre supérieur de plaques) qui sont disposées l'une au-dessus de l'autre, avec un espace entre plaques préservé par des cales 8.

Les plaques 6 et 7 sont dotées de perforations 6a et 7a et sont positionnées de sorte que ces perforations soient décalées d'une plaque par rapport à la plaque voisine.

La densité de répartition des plaques est telle que le pourcentage de sections ouvertes soit compris entre 0,1 et 10% et, en particulier égale à 5% dans les exemples décrits plus loin. Par pourcentage de sections ouvertes on entend, selon l'habitude, le rapport dans une section droite de la somme des surfaces ouvertes à la surface utile de la plaque.

En outre, les perforations de chaque plaque sont prévues de façon que chacune d'elles ait un diamètre supérieur à 300 µm. Au-dessus de ce seuil le diamètre des perforations est adapté à la granulométrie moyenne $G_m$ de la résine utilisée: une valeur de l'ordre de 4 $G_m$ à 6 $G_m$ paraît donner les meilleurs résultats. Par exemple, pour les résines «Sphérosil» visées aux exemples, on a utilisé des plaques ayant des perforations de diamètre égal à 1 mm.

Les plaques 6 et 7 sont maintenues en bordure par des brides annulaires boulonnées 9. Les éléments cylindriques 3 ou 4 ou embase 2 sont collés à leur périphérie sur les surfaces internes annulaires de ces brides. De plus, des joints annulaires tels que 10 assurent l'étanchéité périphérique, d'une part entre plaques, d'autre part entre brides

et plaques.

La solution (solution à traiter au cours de la phase de fixation, solution d'eau distillée au cours des lavages ou solution de régnénération au cours de la phase d'élution) pénètre dans l'embase 2 par un conduit 11. Pour assurer un débit rigoureusement constant, ces solutions sont disposées dans des réservoirs 12 (solution à traiter ou solution de régénération) et 13 (solution d'eau distillée) qui sont disposées à une hauteur appropriée au-dessus de la colonne 1 pour délivrer le débit désiré. Un débitmètre 14 et un diaphragme 15 permettent de réaliser un réglage fin de ce débit.

La solution à traiter est stockée dans un bac 16 où elle est agitée et maintenue à température régulée. La solution de régénération est stockée dans les mêmes conditions dans un bac 17. L'eau distillée est stockée dans un bac 18. La solution en sortie de colonne peut être stockée dans un bac 19. Une pompe 20 et un système de conduits et vannes permet d'alimenter à chaque phase les réservoirs 12 ou 13, en solutions appropriées à partir des bacs 16–18, de sorte que le niveau des réservoirs soit sensiblement constant.

Le système ci-dessus décrit assure une alimentation de la colonne 1 avec un débit dont les variations sont en pratique inférieure à ∓5%.

En sortie de la colonne 1, le liquide est recueilli par surverse en partie haute de l'étage supérieur pour être éventuellement stocké dans le bac 19.

Par ailleurs, la figure 4 schématise une installation modifiée par rapport à la précédente pour autoriser un fonctionnement en continu. La colonne, ses systèmes perforés de distribution et les moyens d'alimentation en liquide à débit constant sont similaires.

Toutefois, dans cette installation, la résine chromatographique est amenée à circuler en continu d'un étage à l'étage inférieur et les phases de lavage et d'élution sont effectuées à l'extérieur de la colonne, cette dernière réalisant la fixation chromatographique en continu.

Les aménagements effectués sur l'installation sont les suivants.

Comme le représente la figure 5, entre deux étages superposés de la colonne, est disposé un tube 21 de passage de résine qui traverse le système perforé de distribution. Ce tube s'étend entre la partie haute 21a du lit de l'étage du dessus et la partie basse 21b du lit de l'étage du dessous. Ainsi la résine peut circuler par surverse dans les divers tubes 21, successivement d'un étage à celui du dessous.

Pour autoriser cette surverse, chaque tube de passage 21 dépasse, à chaque étage, d'une hauteur $H_B$ inférieure à la hauteur $H_S$ de la portion non occupée de l'étage considéré. Ainsi, le lit de résine fluidisé qui se forme à l'intérieur de chaque tube 21 ne déborde pas du tube à l'étage du dessus, ce qui constitue la condition de circulation par gravité de la résine d'un étage à l'étage inférieur.

De plus, à chaque étage, une chicane 22 est disposée entre les deux orifices des deux tubes 21 qui débouchent à l'étage considéré (orifice inférieur 21'b du tube communiquant avec l'étage du dessus et orifice supérieur 21a du tube communiquant avec l'étage du dessous). Chaque chicane 22 peut être constituée par un manchon tubulaire entourant le tube 21; ce manchon se prolonge en partie haute au-dessus du niveau de l'orifice 21a et préserve en partie basse un passage 22a pour la résine.

La résine provenant de l'étage du dessus et débouchant par l'orifice 21'b est moins chargée et donc généralement plus légère et a tendance à monter dans le lit; la chicane 22 évite que se crée un court-circuit de résine entre les orifices 21'b et 21a: elle supprime les zones mortes du lit où la résine ne serait pas renouvelée et assure ainsi un renouvellement complet de celle-ci (il est à noter que, pour certaines résines et certaines macromolécules à fixer, la masse volumique de la résine ne change pas au cours de l'échange: dans ce cas, la chicane est moins essentielle mais améliore l'uniformité globale de fixation).

Par ailleurs, en partie basse de la colonne, le tube de passage inférieur 23 traverse l'embase d'alimentation et est associé à des moyens hydrauliques d'entraînement de la résine 24, en l'exemple vers un bac de récupération 25. La résine de ce bac est régénérée dans une colonne externe qui peut également fonctionner en lit fluidisé; l'entraînement vers cette colonne externe peut être assuré en continu.

La résine chromatographique régénérée est disposée dans un réservoir de stockage 26, soit par un transfert continu depuis la colonne de régénération, soit par alimentation manuelle. Ce réservoir hermétiquement fermé est rempli d'eau distillée et peut être alimenté en eau distillée par un conduit 27 comprenant une vanne et un débitmètre.

La résine peut être hydrauliquement transportée à travers un tube 28 vers un tapis roulant 29 qui est associé à une pompe à vide en vue d'éliminer l'eau entourant les grains de résine.

Le tapis 29 déverse la résine régénérée, à un débit ajustable, dans un tube de passage supérieur 21'', muni d'un entonnoir supérieur.

Les exemples 1 et 2 ont été mis en œuvre sur une installation du type de celle représentée à la figure 2 et l'exemple 3 dans une installation du type de celle représentée aux figures 4 et 5.

Exemple 1

La colonne de l'installation comporte trois étages dans cet exemple; la hauteur de chaque étage est de 50 cm, le diamètre de la colonne étant de 5 cm.

Cet exemple vise à extraire les albumines β-lactoglobuline (masse moléculaire sous forme dimère: 34 000) et α-lactalbumine(14 000) d'un lactosérum doux, résidu des fabrications de fromage fourni par les «Fromageries Bel».

La résine chromatographique utilisée est une résine «Sphérosil QMA» fabriquée par «Rhône Poulenc». La distribution granulométrique initiale est de 100 à 300 μm. On procède à un tamisage à sec pour retenir la plage de grains compris entre 200 et 250 μm, soit une granulométrie moyenne

de $G_m$=225 µm. La masse volumique de la résine «Sphérosil QMA» est $M_v$=1400 Kg/m$^3$. Le couple de valeurs ($G_m$, $M_v$) caractérisant ladite résine est représenté au point R sur le diagramme de la figure 1.

Cette résine est une résine chromatographique constituée de grains sphériques de silice, possédant une surface spécifique de 25 m$^2$/g, un diamètre poreux moyen de 125 nm (1250 Å) et un volume poreux de 1 cm$^3$/g, revêtus d'un polymère réticulé styrène vinyl triethoxysilane, portant les groupements fonctionnels:

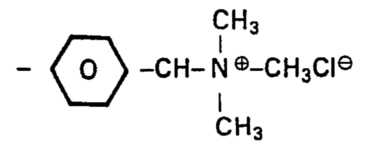

La vitesse minimale de fluidisation d'une telle résine est $V_{mf}$ = 1,4·10$^{-4}$ m/s.

Le diamètre moyen des sections ouvertes du distributeur de chaque étage de la colonne correspond dans ces conditions à environ: 4 $G_m$.

La quantité de résine déposée à chaque étage est de 130 cm$^3$.

La colonne est alimentée à sa base par le lactosérum, avec un débit de solution égal à 9 l/h ne variant pas de plus de 5% par rapport à cette valeur nominale grâce à la nature des moyens d'alimentation prévus (bacs en charge à hauteur déterminée).

Ce débit, dans une colonne de section 1,96·10$^{-3}$ m$^2$ correspond à une vitesse de liquide égale à 1,3·10$^{-3}$ m/s, soit 9 $V_{mf}$.

Lorsque le débit est établi, on constate que le lit dans chaque étage subit une expansion de 64% et présente une hauteur de 20 cm. On constate visuellement une bonne fluidisation stable.

A son entrée le lactosérum présente les concentrations suivantes: 3,0 g/l en β-lactoglobuline et 1,2 g/l en α-lactalbumine.

Le procédé est mis en œuvre pendant deux heures. La figure 7 représente les courbes donnant, en fonction du temps, le rapport des concentrations à la sortie de chaque étage C à la concentration initiale Co; les courbes $\alpha_1$, $\alpha_2$ et $\alpha_3$ représentent la variation de $\frac{C}{Co}$ pour l'α-lactalbumine, respectivement à la sortie de l'étage inférieur, intermédiaire et supérieur. Les courbes $\beta_1$, $\beta_2$ et $\beta_3$ représentent la variation de $\frac{C}{Co}$ pour la β-lactoglobuline respectivement à la sortie de l'étage inférieur, intermédiaire et supérieur. Les mesures ont été faites sur des prélèvements effectués toutes les 3 minutes, par électrophorèse de zone sur membrane en acétate de cellulose.

On constate que pendant 30 minutes la β-lactoglobuline et l'α-lactalbumine sont totalement fixées sur la résine (palier initial des courbes $\alpha_3$ et $\beta_3$) puis que seule la β-lactoglobuline continue d'être adsorbée pendant une quinzaine de minutes alors que l'α-lactalbumine se trouve dans le courant de sortie de la colonne.

Lorsque l'opération de fixation est terminée ($C/C_o$=1), on procède à un lavage à l'eau distillée pendant 20 minutes environ.

La résine est alors éluée à l'aide d'une solution d'acide chlorhydrique 0,1 N pendant 2 heures.

On procède alors à un nouveau lavage à l'eau distillée pendant 1 heure environ et la colonne est prête pour un nouveau cycle.

Pendant toutes ces opérations (lavage, élution), la résine de la colonne est fluidisée de façon analogue.

La quantité de protéines issue de la phase d'élution est, pour la β-lactoglobuline, 10,5 g ce qui représente une fixation de 102 mg par gramme de résine sèche et, pour l'α-lactalbumine, 1,7 g ce qui représente 16,5 mg par gramme de résine sèche. L'éluat contient donc 86,1% de β-lactoglobuline et 13,9% d'α-lactalbumine. Or, les proportions relatives de ces protéines dans le produit de départ (lactosérum) étaient de 71% en β-lactoglobuline et 29% en α-lactalbumine. Il y a donc un enrichissement en β-lactoglobuline.

Le produit d'élution contenant ces protéines est débarrassé du lactose, fines de caséine et sels minéraux initialement contenus dans le lactosérum, les protéines étant pures et dans leur état natif (non dénaturées).

Les capacités de fixation de la résine «Sphérosil QMA» en couche fluidisée de trois étages sont sensiblement identiques à celles obtenues à l'aide d'une couche fixe lors de travaux antérieurs, voir les publications suivantes:

– SKUDDER P.J. «Recovery and Fractionation of Proteins from Cheese whey using a Porous Silica – Based Ion Exchange Medium» Chemistry and Industry, 21, 810–814 (1983),

– BOURGEOIS C.M. et LEROUX P. «Protéines Animales (extraits concentrés et isolats en alimentation humaine), chap. 3, Collection Sciences Techniques agro-alimentaires, Lavoisier (DR).

Les essais ci-dessus décrits ont été renouvelés des dizaines de fois, sans que l'on constate un colmatage quelconque, que ce soit au niveau de la couche ou à celui des distributeurs; il est à noter que le lactosérum utilisé était à l'état brut et contenait des impuretés solides en suspension.

De plus, aucune diminution d'efficacité de la résine n'a pu être observée, ni aucune usure de celle-ci.

Exemple 2

Cet exemple est mis en œuvre dans les mêmes conditions que l'exemple 1, à l'exception de la quantité de résine déposée sur chaque étage qui est de 110 cm$^3$.

Lorsque le débit est établi, on constate que le lit dans chaque étage subit une expansion de 69% et présente une hauteur de 18 cm. On constate visuellement une bonne fluidisation stable.

A son entrée, le lactosérum présente les mêmes concentrations que dans l'exemple 1 (3,0 g/l en β-lactoglobuline et 1,2 g/l en α-lactalbumine).

Le procédé est mis en œuvre pendant 2 heures. La figure 8 présente les courbes donnant, en

fonction du temps, le rapport des concentrations à la sortie de chaque étage C à la concentration initiale Co.

On constate que la quantité de résine sur chaque étage étant moins importante que dans l'exemple 1, les durées pendant lesquelles la totalité des deux protéines d'une part, puis la β-lactoglobuline seule d'autre part, sont adsorbées, sont plus courtes que dans l'exemple 1 (d'une quinzaine de minutes chacune).

On procède à la succession de lavage, élution, lavage de la même façon que dans l'exemple 1.

La quantité de protéines obtenue par élution correspond à une fixation de 110 mg par gramme de résine sèche pour la β-lactoglobuline et 19,5 mg par g de résine sèche pour l'α-lactalbumine. L'éluat contient donc 84,9% de β-lactoglobuline et 15,1% d'α-lactalbumine. Aux erreurs expérimentales et analytiques près, ces proportions sont analogues à celles observées pour l'exemple 1.

Exemple 3

Cet exemple a été mis en œuvre dans l'installation décrite précédemment et représentée aux figures 4 et 5, la colonne comportant trois étages de 60,5 cm de hauteur chacun.

Cet exemple vise à extraire les albumines β-lactoglobuline et α-lactalbumine d'un lactosérum doux, résidu des fabrications de fromages, fourni par les «Fromageries Bel».

La résine utilisée («Sphérosil QMA») présente les mêmes caractéristiques que dans l'exemple 1.

Le diamètre moyen des sections ovetes du distributeur de chaque étage de la colonne est 4 $G_m$.

Les tubes de jonction servant à véhiculer la résine de haut en bas de la colonne ont un diamètre intérieur de 1,4 cm et dépassent à chaque étage d'une hauteur $H_B$=25 cm.

Le débit de résine introduit dans la colonne est maintenu à 0,14 l/h.

Le système d'évacuation de la résine à la base de la colonne comprend trois vannes électromagnétiques contrôlées par un temporisateur. Les particules de solide traversent la vanne supérieure ouverte et s'accumulent dans un tube en plastique souple. Toutes les 20 secondes, la vanne supérieure est fermée, alors que la vanne latérale qui s'ouvre en même temps que la vanne inférieure injecte de l'eau distillée qui entraîne les particules vers le bas.

La colonne est alimentée à sa base par le lactosérum, avec un débit de solution égal à 9 l/h, ce débit étant contrôlé de la même façon que dans l'exemple 1.

Lorsque le débit est établi, on constate que le lit dans chaque étage présente une hauteur de 25 cm correspondant à la hauteur du dépassement $H_B$ du tube de jonction sur l'étage considéré.

Chaque étage a une hauteur de 60,5 cm de telle sorte que la hauteur de la couche fluidisée retenue dans le tube de jonction de l'étage possède un niveau inférieur à celui de la couche fluidisée de l'étage immédiatement supérieur, empêchant ainsi le débordement de la résine dans l'étage du dessus.

On constate visuellement une bonne fluidisation stable et une bonne circulation des deux phases solide et liquide.

A son entrée, le lactosérum présente les concentrations suivantes: 3,0 g/l en β-lactoglobuline et 1,2 g/l en α-lactalbumine.

Le procédé fonctionne en continu mais, pour l'exemple considéré, il est mis en œuvre pendant 3 heures. La figure 9 représente les courbes donnant en fonction du temps, le rapport des concentrations à la sortie de chaque étage C à la concentration initiale Co.

On constate que l'opération comporte une phase transitoire avant d'atteindre le régime stationnaire de fonctionnement.

On note que l'α-lactalbumine ($\alpha_1$, $\alpha_2$, $\alpha_3$) est retenue en moins grande quantité sur la résine que la β-lactoglobuline ($\beta_1$, $\beta_2$, $\beta_3$).

A la sortie de la colonne (troisième étage) le liquide contient environ 50% d'α-lactalbumine initiale alors qu'il ne contient plus que 25% de la β-lactoglobuline, le reste étant fixé sur la résine.

## Revendications

1. Procédé de séparation chromatographique de macromolécules biologiques de poids moléculaire supérieur à 5000, contenues dans une solution, du type consistant à amener la solution au contact d'un lit de résines, chromatographiques sélectives, spécifiques des macromolécules à séparer en vue de réaliser une adsorption sélective, ledit procédé étant caractérisé en ce que:

– l'on utilise une résine chromatographique ayant une granulométrie moyenne $G_m$ et une masse volumique $M_v$ dont les valeurs sont situées dans la zone Z (non hachurée) du diagramme de la figure 1,

– l'on dispose ladite résine chromatographique dans une colonne comprenant au moins un étage pourvu à sa base d'un système perforé de distribution présentant les caractéristiques suivantes:

– pourcentage de sections ouvertes compris entre 0,1% et 10%,

– diamètre moyen des sections ouvertes supérieur à environ 300 µm,

– diamètre moyen des sections ouvertes compris entre 2 $G_m$ et 20 $G_m$,

– et l'on alimente le ou chaque étage de la colonne à travers son système de distribution au moyen de la solution de manière à fluidiser le lit de résine chromatographique, en ajustant le débit de la solution de façon à obtenir dans la colonne une vitesse linéaire du liquide (ramenée à la section droite totale) comprise entre 1,5 $V_{mf}$ et 12 $V_{mf}$, où $V_{mf}$ est la vitesse minimale de fluidisation de la résine.

2. Procédé selon la revendication 1, dans lequel la résine chromatographique est disposée dans une colonne sensiblement verticale comprenant entre 2 et 5 étages superposés, parcourus successivement par la solution, le soutirage de ladite solution étant effectué par surverse en partie haute de l'étage supérieur, chaque étage (à l'exception de ce dernier) étant délimité par deux systèmes

perforés de distribution), l'un distribuant la solution à la base de l'étage considéré, l'autre distribuant la solution vers l'étage situé immédiatement au-dessus.

3. Procédé selon l'une des revendications 1 ou 2, caractérisé en ce que la colonne est alimentée de sorte que le débit de solution à l'intérieur de celle-ci soit sensiblement constant au moins pendant les phases de fixation sélective, la variation de ce débit par rapport à sa valeur nominale étant inférieure à ∓5%.

4. Procédé selon l'une des revendications 1, 2 ou 3, caractérisé en ce que l'on réalise chaque système perforé de distribution au moyen d'au moins deux plaques perforées (6, 7) superposées avec un espace entre plaques, lesdites plaques étant dotées de perforations (6a, 7a) et étant positionnées de façon à décaler les perforations d'une plaque par rapport à celles de la ou des plaques voisines (Fig. 3).

5. Procédé selon l'une des revendications 1, 2, 3 ou 4, dans lequel la résine chromatographique est enfermée à chaque étage de la colonne en l'absence de circulation d'un étage à l'autre, la séparation en lit fluidisé étant effectuée par cycles de fixation sélective, lavage, élution et lavage dans la colonne.

6. Procédé selon l'une des revendications 1, 2, 3 ou 4, dans lequel la résine chromatographique est amenée à circuler en continu de haut en bas dans la colonne, avec une alimentation continue en résine en partie haute et un soutirage continu en partie basse, la séparation en lit fluidisé étant effectuée en continu dans la colonne, avec exécution concomitante d'opérations de lavage, élution et lavage à l'extérieur de la colonne.

7. Procédé selon les revendications 2 et 6 prises ensemble, dans lequel l'on dispose entre deux étages superposés de la colonne, un tube (21) de passage de résine, traversant le système de distribution et s'étendant entre la partie haute (21a) du lit de l'étage du dessus et la partie basse (21b) du lit de l'étage du dessous, la résine circulant par surverse dans ledit tube, de l'étage du dessus vers l'étage du dessous (Fig. 5).

8. Procédé selon la revendication 7, dans lequel, à chaque étage, l'on dispose dans le lit de résine au moins une chicane (22) disposée entre l'orifice (21'b) du tube communiquant avec l'étage du dessus et l'orifice (21a) du tube communiquant avec l'étage du dessous, de façon à assurer un renouvellement complet de la résine de l'étage (Fig. 5).

9. Procédé selon l'une des revendications 7 ou 8, dans lequel les tubes de passage sont disposés dans la colonne de sorte que le lit de résine fluidisé formé dans un tube à un étage donné ne déborde pas du tube à l'étage du dessus.

10. Procédé selon l'une des revendications 1 à 9, pour la séparation chromatographique des protéines d'un lactosérum.

11. Procédé selon la revendication 10 pour l'extraction des albumines β-lactoglobulines et α-lactalbumines d'un lactosérum, dans lequel on utilise une résine chromatographique du type «Sphérosil» de granulométrie $G_m$ comprise entre 75 et 300 μm, ladite résine étant préalablement tamisée de sorte que la dispersion des tailles de grains autour de cette valeur moyenne soit au plus égale à 50%.

**Patentansprüche**

1. Verfahren zur chromatographischen Trennung von biologischen Makromolekülen mit einem Molekulargewicht, das höher ist als 5000 und die in einer Lösung enthalten sind, und zwar derart, dass die Lösung mit einem Bett von chromatographisch selektiven und den zu trennenden Makromolekülen spezifischen Harzen in Kontakt gebracht wird, um selektive Adsorption zu bewirken, wobei das besagte Verfahren dadurch gekennzeichnet ist,

– dass man von einem chromatographischen Harz mit einer mittleren Korngrösse $G_m$ und einer volumetrischen Masse $M_v$ Gebrauch macht, deren Werte innerhalb des Bereiches Z (nicht schraffiert) des Diagramms in Bild 1 liegen,

– dass man das besagte chromatographische Harz in einer Säule anordnet, die mindestens eine an ihrem Boden mit einem gelochten Verteilungssystem versehene Stufe umfasst und die folgenden Merkmale besitzt:

– Prozentsatz offener Querschnitte zwischen 0,1% und 10%,

– mittlerer Durchmesser der offenen Querschnitte grösser als etwa 300 μm,

– mittlerer Durchmesser der offenen Querschnitte zwischen 2 $G_m$ und 20 $G_m$

– sowie darin, dass man die oder jede Stufe der Säule mit Hilfe der Lösung durch ihr Verteilungssystem hindurch speist, indem man das Bett chromatographischen Harzes verflüssigt, wobei man die Durchflussgeschwindigkeit der Lösung so regelt, dass in der Säule eine lineare Flüssigkeitsgeschwindigkeit (im Verhältnis zu dem Gesamtquerschnitt) erhält, die zwischen 1,5 $V_{mf}$ und 12 $V_{mf}$ liegt, wobei $V_{mf}$ die Mindestgeschwindigkeit der Harzverflüssigung ist.

2. Verfahren nach Anspruch 1, bei dem das chromatographische Harz in einer im wesentlichen senkrechten Säule angeordnet ist, die von 2 bis 5 übereinander angeordnete Stufen umfasst, wobei die Lösung der Reihe nach durch die besagten Stufen fliesst und die besagte Lösung durch Überlauf im oberen Teil der obersten Stufe abgezogen wird, und zwar ist jede Stufe (mit Ausnahme dieser letzteren) durch zwei gelochte Verteilungssysteme begrenzt, von denen die eine die Lösung am unteren Ende der betreffenden Stufe verteilt, während die andere die Lösung an die unmittelbar darüber befindliche Stufe verteilt.

3. Verfahren nach einem der Ansprüche 1 oder 2, dadurch gekennzeichnet, dass die Säule so gespeist wird, dass die Durchflussgeschwindigkeit der Lösung im Inneren derselben im wesentlichen konstant ist, mindestens während der Phasen selektiver Fixierung, wobei die Schwankung der besagten Durchflussgeschwindigkeit im Verhältnis zu ihrem Nennwert geringer ist als ∓5%.

4. Verfahren nach einem der Ansprüche 1, 2

oder 3, dadurch gekennzeichnet, dass man jedes gelochte Verteilungssystem durch mindestens zwei gelochte Platten (6, 7) erzeugt, die unter Einschaltung eines Zwischenraums zwischen den Platten überlagert sind, wobei die besagten Platten Lochungen (6a, 7a) besitzen und so angeordnet sind, dass die Lochungen der einen Platte im Verhältnis zu denen der benachbarten Platte bzw. Platten versetzt sind (Bild 3).

5. Verfahren nach einem der Ansprüche 1, 2, 3 oder 4, bei dem das chromatographische Harz in jeder Stufe der Säule ohne Zirkulation von einer Stufe zur anderen eingeschlossen ist, wobei die Trennung in dem verflüssigten Bett durch Zyklen selektiver Fixierung, Waschung, Elution und Waschung innerhalb der Säule durchgeführt wird.

6. Verfahren nach einem der Ansprüche 1, 2, 3 oder 4, bei dem das chromatographische Harz zu kontinuierlichem Zirkulieren vom oberen zum unteren Ende der Säule veranlasst wird, unter kontinuierlicher Speisung mit Harz im oberen Teil und kontinuierlichem Abzapfen im unteren Teil, wobei die Trennung innerhalb des verflüssigten Betts kontinuierlich innerhalb der Säule bewirkt wird, und zwar in Verbindung mit der Durchführung von Operationen des Waschens, Eluierens und Waschens aussen an der Säule.

7. Verfahren nach den Ansprüchen 2 und 6 gemeinsam, bei dem zwischen zwei übereinander befindlichen Stufen der Säule ein Harzdurchgangsrohr (21) vorgesehen ist, das durch das Verteilungssystem hindurchführt und sich zwischen dem oberen Teil (21a) des Betts der oberen Stufe und dem unteren Teil (21b) des Betts der unteren Stufe erstreckt, wobei das Harz durch Überlauf innerhalb des besagten Rohrs von der oberen Stufe zu der unteren Stufe zirkuliert (Bild 5).

8. Verfahren nach Anspruch 7, bei dem in jeder Stufe innerhalb des Harzbettes mindestens ein Schirm (22) vorgesehen ist, der sich zwischen der Öffnung (21'b) des mit der oberen Stufe in Verbindung stehenden Rohrs und Öffnung (21a) des mit der unteren Stufe in Verbindung stehenden Rohrs befindet, so dass vollständige Erneuerung des Harzes der Stufe gewährleistet ist (Bild 5).

9. Verfahren nach einem der Ansprüche 7 oder 8, bei dem die Durchgangsrohre so in der Säule angeordnet sind, dass das verflüssigte innerhalb eines Rohres in einer gegebenen Stufe gebildete Bett des Harzes nicht in der oberen Stufe aus dem Rohr fliesst.

10. Verfahren nach einem der Ansprüche 1 bis 9 zur chromatographischen Trennung der Proteine eines Laktoserums.

11. Verfahren nach Anspruch 10 zur Gewinnung von Albuminen, β-Laktoglobulinen und α-Laktalbuminen eines Laktoserums, wobei von einem chromatographischen Harz der Art «Spherosil» mit einer Korngrösse $G_m$ zwischen 75 und 300 μm Gebrauch gemacht wird, wobei das besagte Harz so vorgesiebt wird, dass die Streuung der Korngrössen um den besagten Mittelwert mindestens 50% gleich ist.

## Claims

1. Process for the chromatographic separation of biological macromolecules with a molecular weight of more than 5,000 and contained in a solution, of the type consisting in causing the solution to come into contact with a bed of chromatographically selective resins specific to the macromolecules which are to be separated, with a view to bringing about selective adsorption, said process being characterised in that

– use is made of a chromatographic resin having a mean granulometric size $G_m$ and a volumetric mass $M_v$, the values of which are situated in zone Z (not hatched) of the diagram in figure 1,

– said chromatographic resin is located in a column comprising at least one stage provided at its base with a perforated distribution system with the following characteristics:

– percentage of open sections between 0.1% and 10%,

– mean diameter of the open sections greater than about 300 μm,

– mean diameter of the open sections between 2 $G_m$ and 20 $G_m$

– and the or each stage of the column is fed through its distribution system with the aid of the solution by fluidising the bed of chromatographic resin, the rate of flow of the solution being adjusted so as to achieve within the column a linear velocity of liquid flow (in relation to the total cross-section) between 1.5 $V_{mf}$ and 12 $V_{mf}$, where $V_{mf}$ is the minimum rate of resin fluidisation.

2. Process according to claim 1, in which the chromatographic resin is located within a substantially vertical column comprising between 2 and 5 superimposed stages, the solution flowing successively through said stages and drawing off of said solution being effected by overflow in the high part of the upper stage, each stage (with the exception of this latter one) being bounded by two perforated distribution systems, one distributing the solution to the bottom of the stage in question, and the other distributing the solution toward the stage immediately above.

3. Process according to one of claims 1 or 2, characterised in that the column is supplied in such a way that the rate of flow of the solution within said column is substantially constant, at least during the phases of selective fixation, the variation of this flow rate relative to its nominal value being less than ∓5%.

4. Process according to one of claims 1, 2 or 3, characterised in that each perforated distribution system is produced by means of at least two perforated plates (6, 7) superimposed with a space between the plates, said plates being provided with perforations (6a, 7a) and so positioned as to offset the perforations of one plate in relation to those of the neighbouring plate or plates (fig. 3).

5. Process according to one of claims 1, 2, 3 or 4, in which the chromatographic resin is enclosed within each stage of the column there being no

circulation from one stage to the other, separation in the fluidised bed being effected by cycles of selective fixation, washing, elution and washing in the column.

6. Process according to one of claims 1, 2, 3 or 4, in which the chromatographic resin is caused to circulate continuously from the top to the bottom within the column, with continuous resin feeding at the high part and continuous drawing off at the low part, the separation in the fluidised bed being effected continuously within the column, in conjunction with the carrying out of operations of washing, elution and washing at the outside of the column.

7. Process according to claims 2 and 6 jointly, in which a tube (21) for the passage of resin is provided between two superimposed stages of the column, said tube passing through the distribution system and extending between the high part (21a) of the bed of the upper stage and the low part (21b) of the bed of the lower stage, the resin circulating by overflow in said tube from the upper stage toward the lower stage (fig. 5).

8. Process according to claim 7, in which one provides, in each stage, at least one screen (22) within the resin bed, said screen being located between orifice (21'b) of the tube communicating with the upper stage and orifice (21a) of the tube communicating with the lower stage, so as to ensure complete renewal of the resin in the stage (fig. 5).

9. Process according to one of claims 7 or 8, in which the passage tubes are provided within the column in such a way that the fluidised bed of resin formed within a tube of a certain stage does not flow from the tube at the upper stage.

10. Process according to one of claims 1 to 9 for chromatographic separation of the proteins of a lactoserum.

11. Process according to claim 10 for extracting albumins, β-lactoglobulins and α-lactalbumins from a lactoserum, in which use is made of a chromatographic resin of the type «Spherosil» with a granulometric size $G_m$ between 75 and 300 μm, said resin being pre-sieved so that the dispersion of grain sizes about this mean value is at the most equal to 50%.

1/8

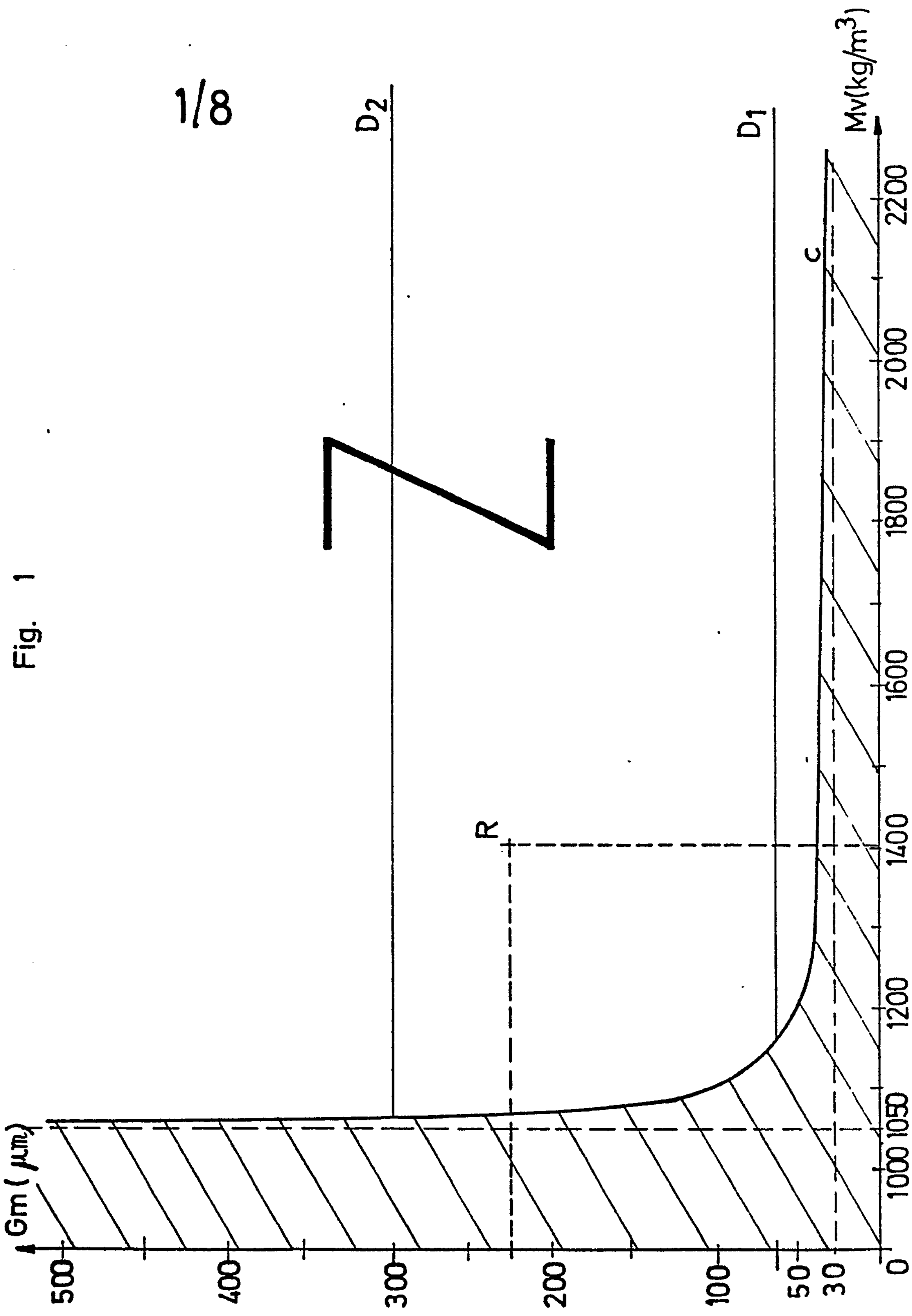
Gm (µm)
Mv(kg/m3)
D2
D1
Z
R
C
500
400
300
200
100
50
30
0
1000
1050
1200
1400
1600
1800
2000
2200

Fig. 1

2/8

Fig. 2

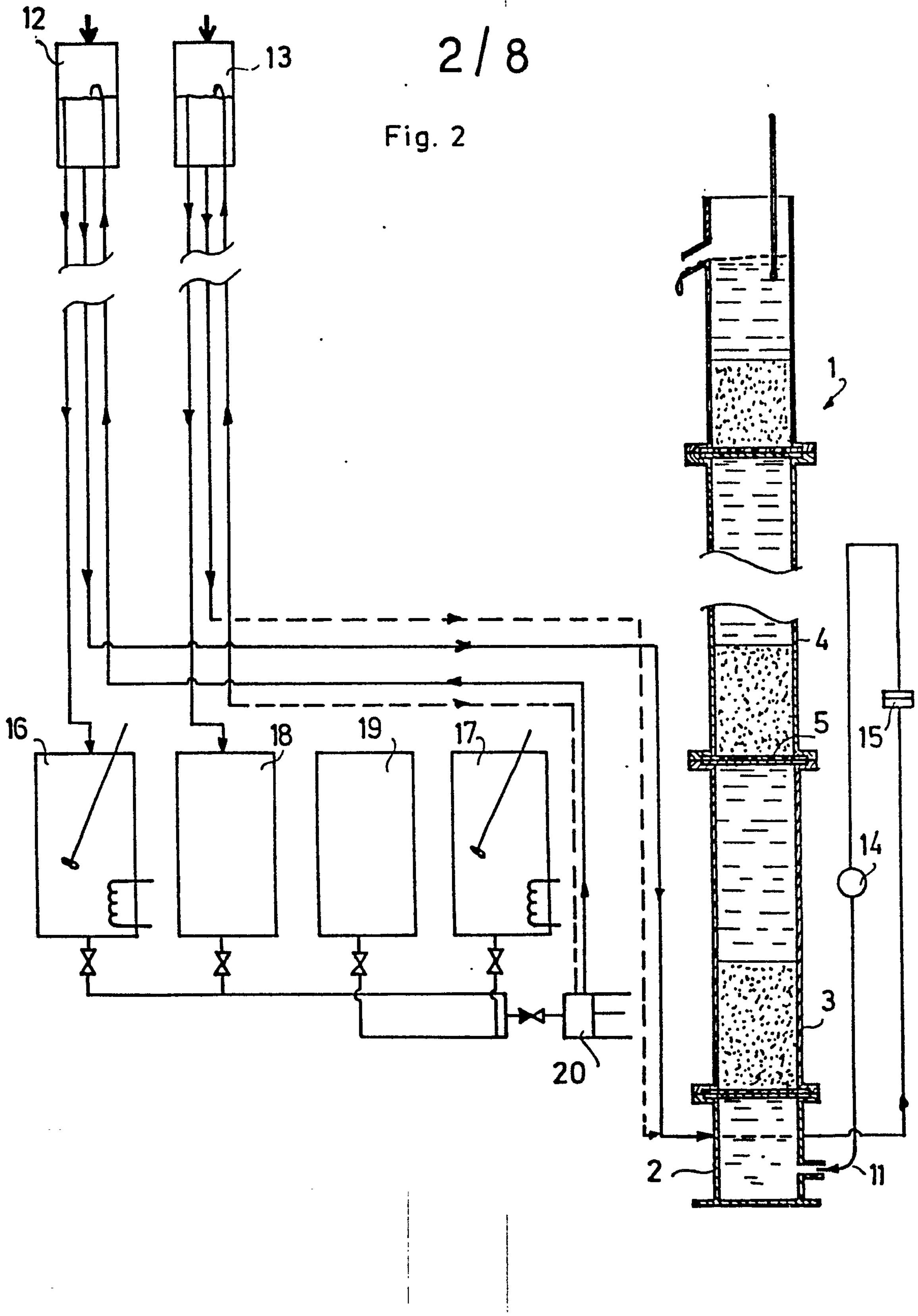

3/8

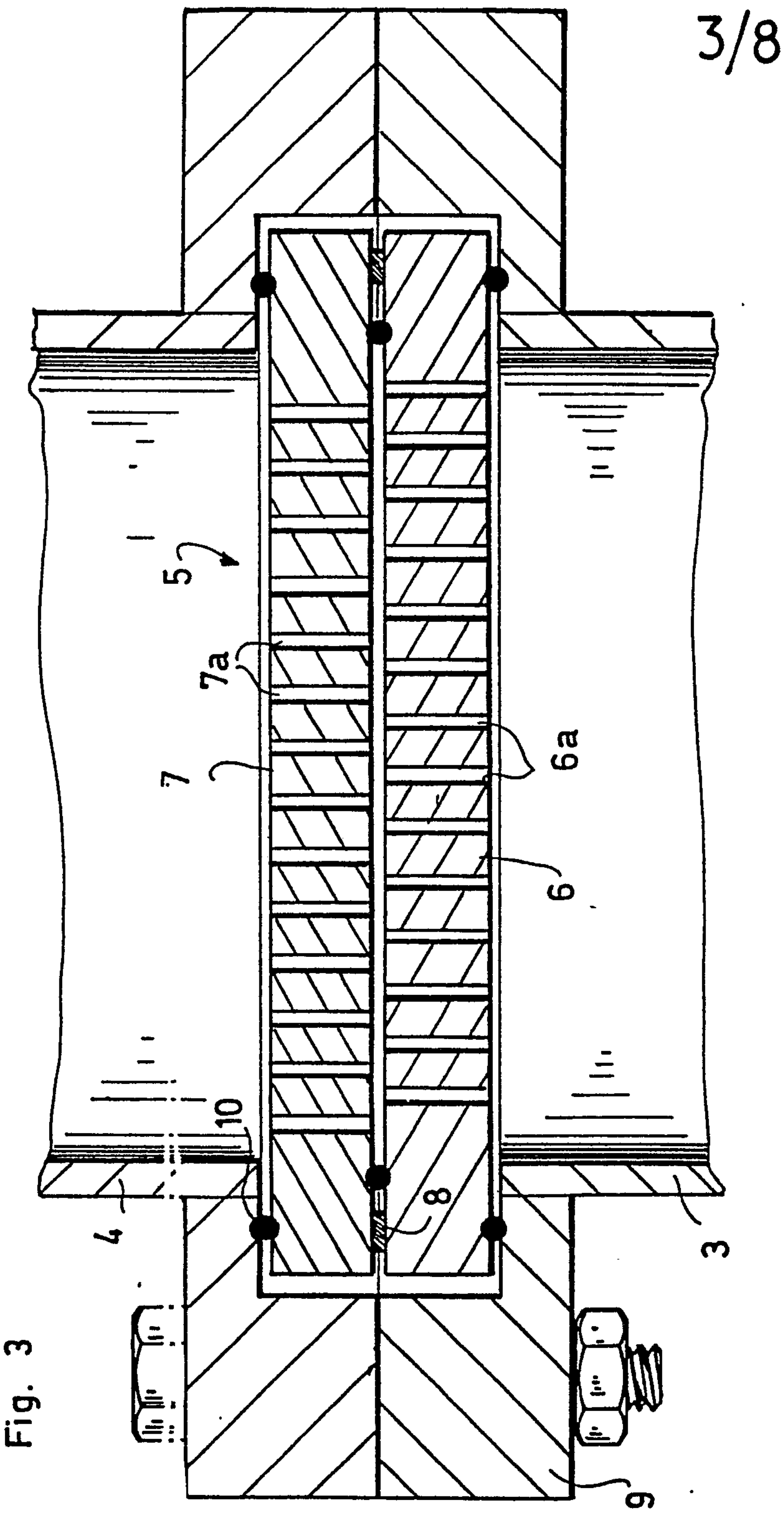


Fig. 3

4/8

Fig. 4

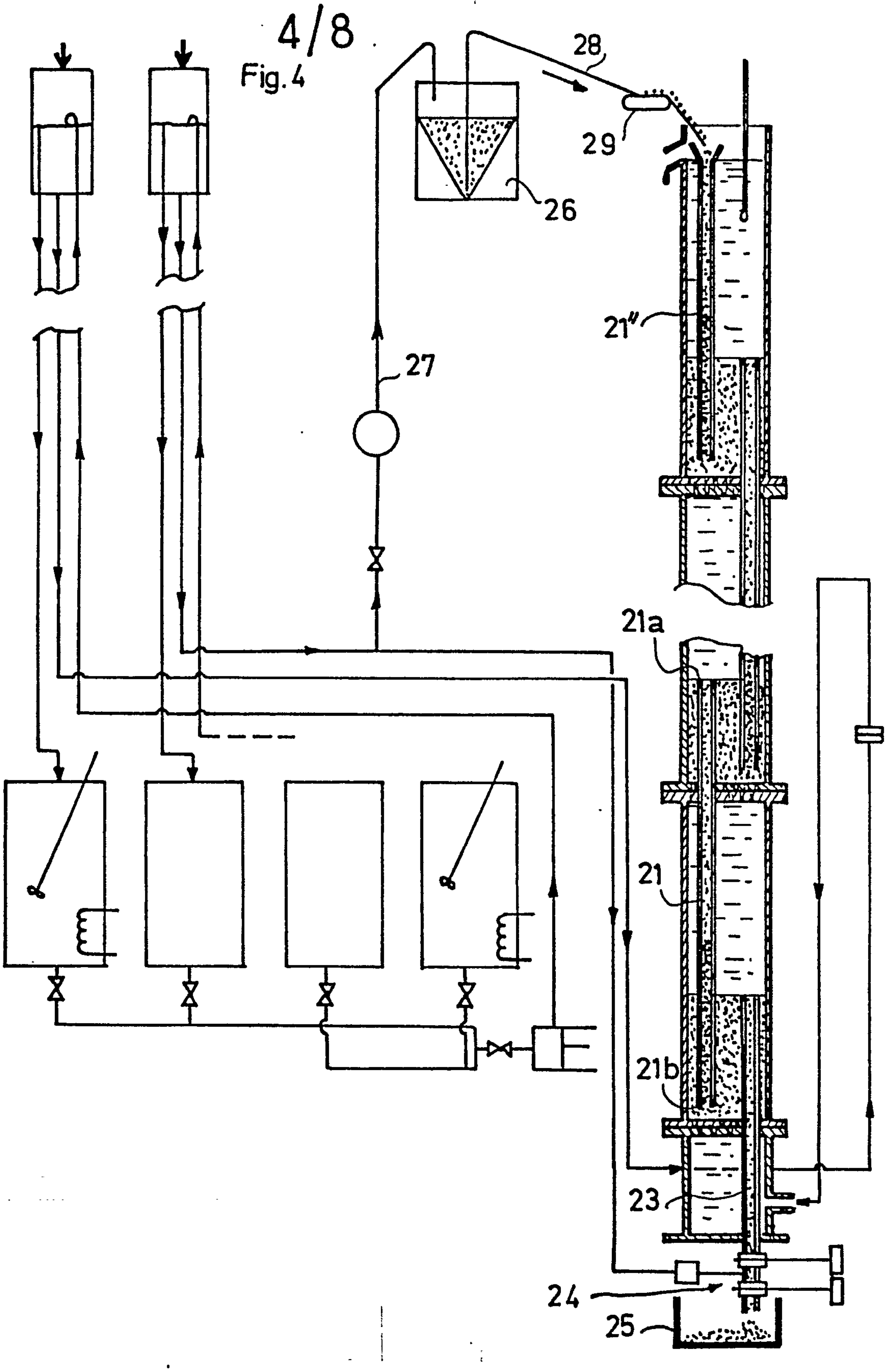

5/8

Fig. 5

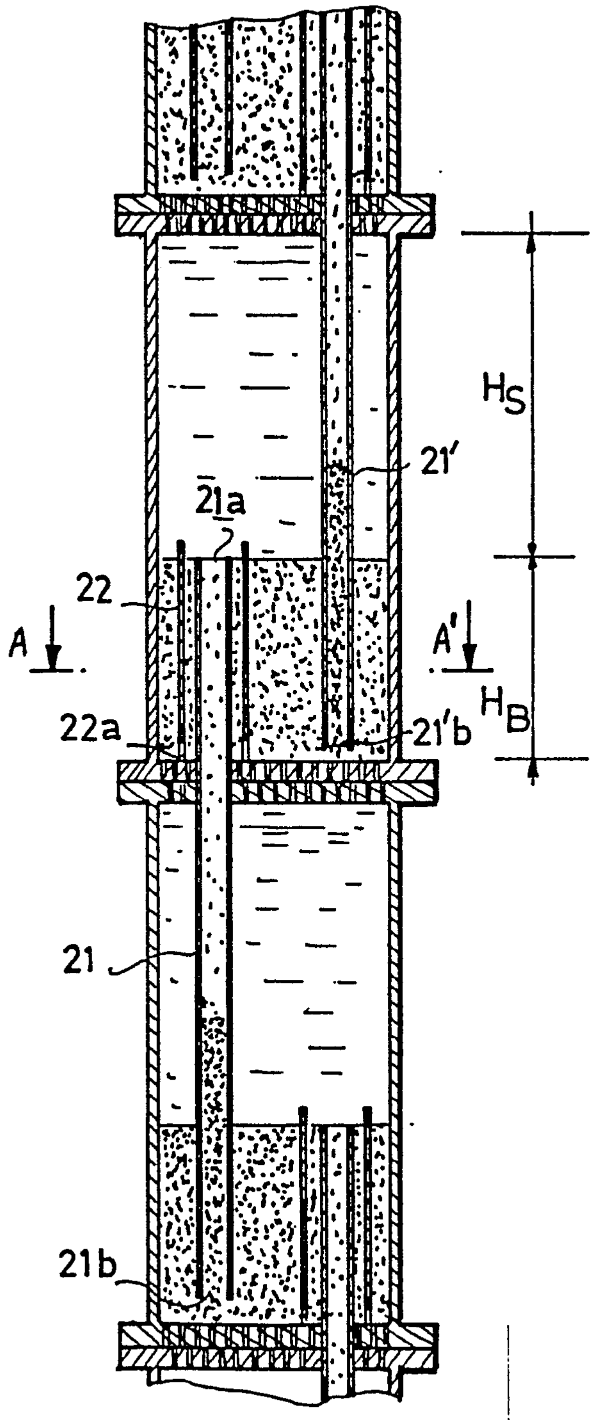


Fig. 6

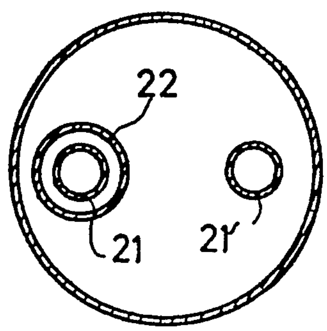

6/8

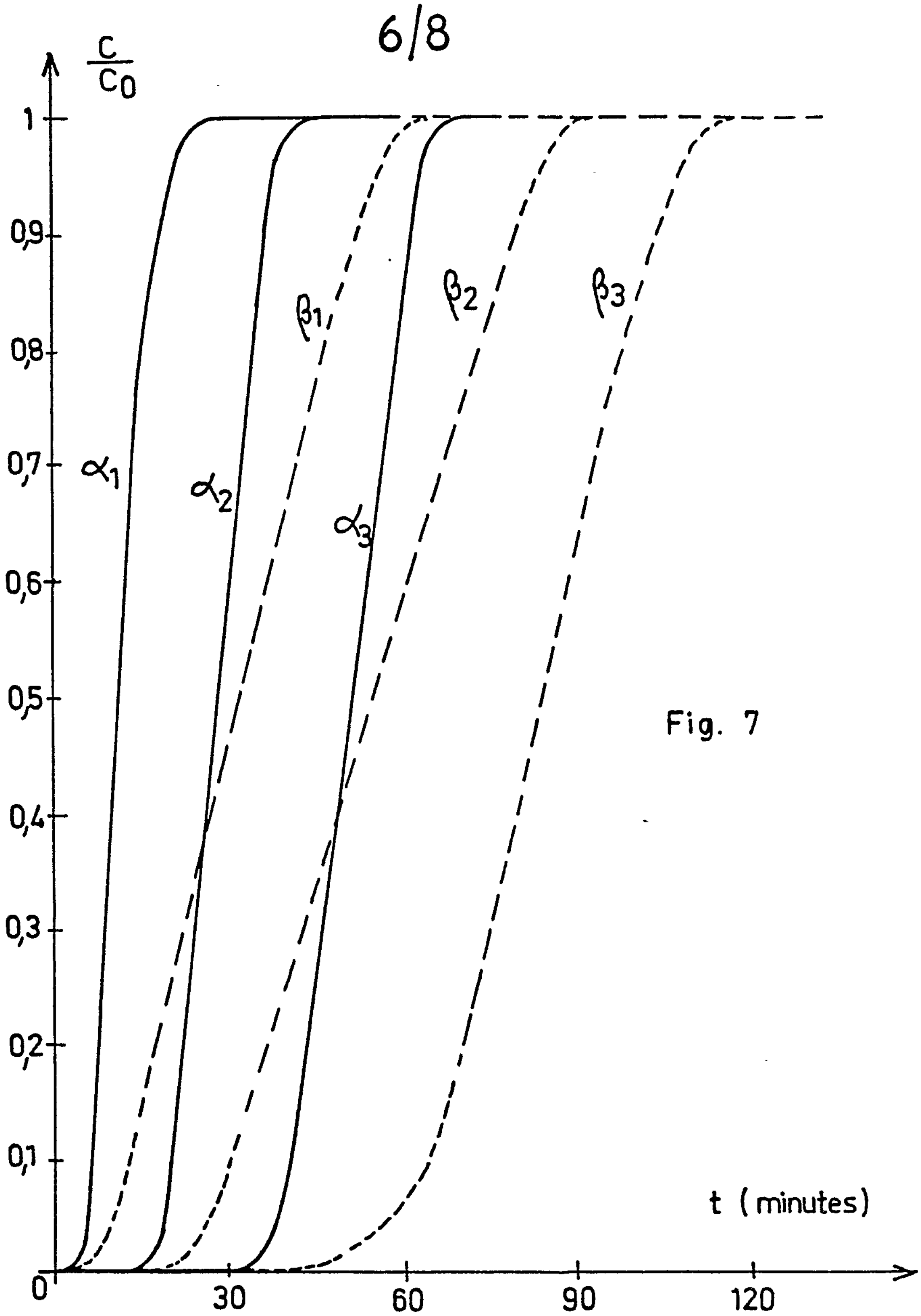

$\frac{C}{C_0}$
1
0,9
0,8
0,7
0,6
0,5
0,4
0,3
0,2
0,1
0
30
60
90
120
t (minutes)
$\alpha_1$
$\alpha_2$
$\alpha_3$
$\beta_1$
$\beta_2$
$\beta_3$


Fig. 7

7/8

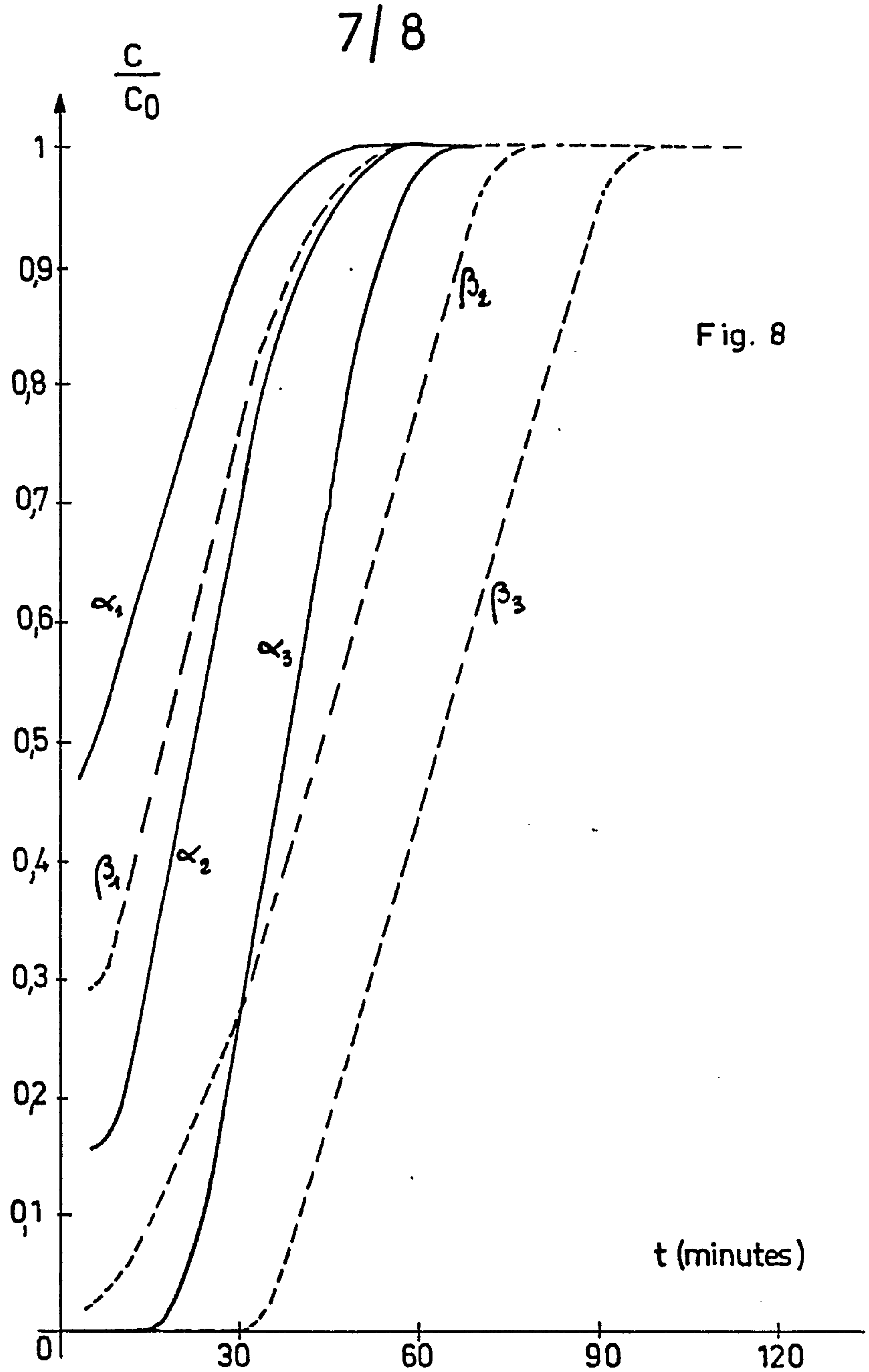
C/C0
1
0,9
0,8
0,7
0,6
0,5
0,4
0,3
0,2
0,1
0
30
60
90
120
t (minutes)
α1
α2
α3
β1
β2
β3

Fig. 8

8/8

Fig. 9

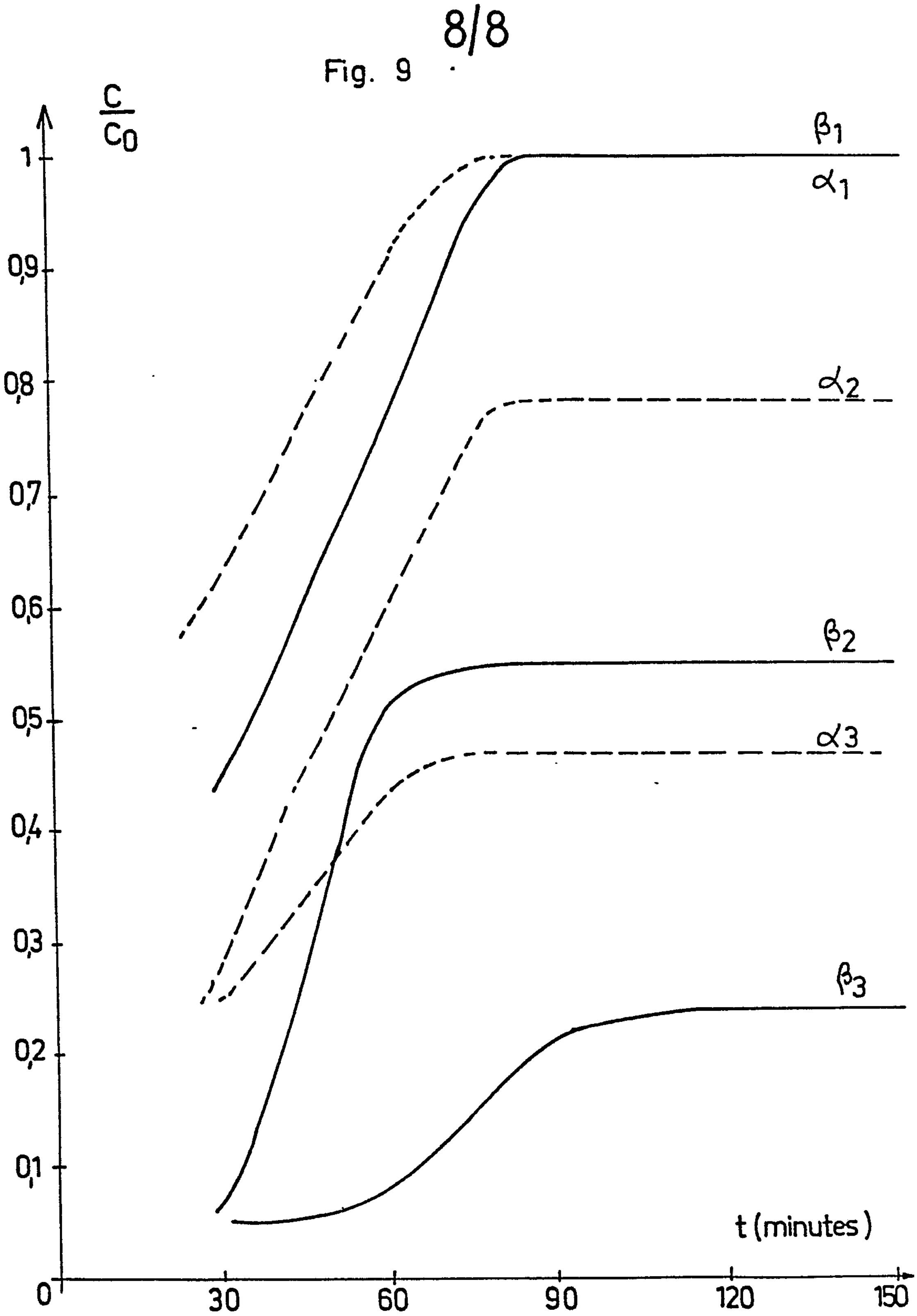
C/C0
1
0,9
0,8
0,7
0,6
0,5
0,4
0,3
0,2
0,1
0
β1
α1
α2
β2
α3
β3
t (minutes)
30
60
90
120
150